# EUROPEAN PATENT SPECIFICATION

(11) **EP 2 274 436 B1**
(45) Date of publication and mention of the grant of the patent: **10.07.2013**
(21) Application number: 09745572.9
(22) Date of filing: 08.05.2009
(51) Int. Cl.: C12P 9/00, C12P 13/06

(54) **Process for preparing phosphatidylserine**
Verfahren zur Herstellung von Phosphatidylserin
Procédé pour la production de phosphatidylsérine

(30) Priority: 15.05.2008 RU 2008119294
(43) Date of publication of application: 19.01.2011
(73) Proprietor: LODERS CROKLAAN B.V., 1521 AZ Wormerveer (NL)
(72) Inventor: TARAN, Viktoria, NL-2628 CV Delft (NL); DRYGIN, Yuri Fedorovich, Moscow 119991 (RU); ZUEVA, Vera Sergeevna, Moscow SP-7, 117997 (RU); GALIULLINA, Raisa Anvarovna, Moscow 117209 (RU)
(74) Representative: Stevens, Ian Edward
(86) International application number: PCT/EP2009/003456
(87) International publication number: WO 2009/138238

(56) References cited:
- EP-A- 1 048 738
- AU-A- 2007 205 762
- SHARMA ET AL: "Purification of phospholipase D from Dacus carota by three-phase partitioning and its characterization" PROTEIN EXPRESSION AND PURIFICATION, vol. 21, 2001, pages 310-316, XP002553607
- HANAHAN ET AL: "A new phospholipide-splitting enzyme specific for the ester linkage between the nitrogenous base and the phosphoric acid grouping" JOURNAL OF BIOLOGICAL CHEMISTRY, vol. 169, 1947, pages 699-705, XP002553608
- DATABASE FSTA INTERNATIONAL FOOD INFORMATION SERVICE (IFIS), FRANKFURT-AM-MAIN, DE; Original article in Japanese, 1998, KAMATA E / JOURNAL OF THE JAPAN OIL CHEMISTS' SOCIETY; 1998, PP. 51-56: "Preparation of phosphatidylserine from soybean phospholipid by cabbage phospholipase D" XP002553697 Database accession no. 1998-10-j2272 FSTA
- QUARLES ET AL: "The distribution of phospholipase D in developing and mature plants" BIOCHEMICAL JOURNAL, vol. 112, 1969, pages 787-794, XP002553609

## Description

The present invention relates to a process for producing a phospholipid. In particular, the invention relates to a process for producing phosphatidylserine using phospholipase D from carrot.

Phosphatidylserine is a phospholipid nutrient found in fish, green leafy vegetables, soybeans and rice, and is essential for the normal functioning of neuronal cell membranes. In apoptosis, phosphatidylserine is transferred to the outer leaflet of the plasma membrane. This is part of the process by which the cell is targeted for phagocytosis. Phosphatidylserine has been used to slow cognitive decline in early-onset Alzheimer's disease. The substance is sold as a dietary supplement to people who believe they can benefit from an increased intake.

The dietary supplement was originally processed from cow brains. Prion disease scares in the 1990s outlawed this process and, as a consequence, a soy-based alternative was developed. Phosphatidylserine sold now is made from plants instead of animals. A drink containing a soy emulsion of phosphatidylserine is marketed under the name *Function Brainiac* to people who feel they have cognitive deficits and would benefit from ingesting the chemical.

The processes for producing and purifying phosphatidylserine typically involve the enzymatic conversion of phosphatidylcholine into phosphatidylserine by a transesterification reaction (also referred to as transphosphatidylation) catalysed by the enzyme phospholipase D, with subsequent purification brought about mainly by extracting the phosphatidylserine with organic solvents.

The conventional processes for synthesising phosphatidylserine by enzymatic conversion use either two-phase systems comprising water and an organic solvent or an aqueous medium, in the presence of phospholipase D obtained from fermentation broths of microorganisms strains which produce extracellular phospholipase D.

For example, EP-A-1048738 describes a process for the preparation of phosphatidylserine by reacting a phospholipid with serine in an aqueous dispersion in the presence of phospholipase D and calcium salts.

US 2004/02355119 discloses a method for the production of phospholipids Involving the use of a phospholipase D.

WO 00/77183 relates to the enzymatic preparation of phospholipids in aqueous media.

The transphosphatidylation reaction which converts phosphatidylcholine into phosphatidylserine competes with the hydrolysis of phosphatidytcholine, which produces phosphatidic acid. As a consequence, this has led to the use of strains of microarganisms which typically have a transphosphatidylation activity higher than the hydrolysing activity.

There remains a need for an economic and effective process for the preparation of phosphatidylserine using phospholipase D.

It has now been surprisingly found that phospholipase D obtained from carrot can be used to produce effectively the transesterification product, phosphatidylserine.

According to a first aspect of the invention there is provided a process for preparing phosphatidylserine comprising reacting a phospholipid with serine In the presence of water, the phospholipase D from carrot and at least one surfactant Thus, the process comprises the enzymatic conversion of the phospholipid into phosphatidylserine by a transesterification reaction catalysed by the enzyme phospholipase D derived from carrot.

In a second aspect of the invention there is provided a process for preparing phosphatidic acid comprising hydrolysing a phospholipid in the presence of water, a phospholipase D from carrot, and at least one surfactant.

Sharma et a/, Protein Expression and Purification, 21, 310-316, (2001) discloses the purification of phospholipase D from carrot by three-phase partitioning.

AU-A-2007 205762 relates to a method for producing phospholipid using transphosphatidylation. The method comprises homogenizing a mixture of raw material phospholipid, a hydroxyl-containing acceptor, phospholipase D and water in the absence of an organic solvent to form a homogenized mixture which is reacted at 15 to 65°C.

The phospholipid that can be used in the processes of the invention can be synthetic or natural. Representative examples of phospholipids that may be used include phosphatidylcholine, phosphatidylethanolamine, phosphatidylinositol, phosphatidyl glycerol, sphingomyelin and mixtures thereof. The preferred phospholipid for use in the processes of the invention is phosphatidylcholine.

The phospholipid is preferably obtained from animal or vegetable sources and may include naturally occurring substances such as lecithin, cephalin, and sphingomyelin. Lecithin is particularly preferred.

Lecithin contains a mixture of glycolipids, triglycerides, and phospholipids (e.g., phosphatidylcholine, phosphatidylethanolamine, and phosphatidylinositol) and may be derived from natural sources or synthetic sources. Preferably, lecithin for use in the processes of the invention is derived from vegetable sources, such as from soy beans, egg yolks or rapeseed, using conventional processing methods. The phospholipid content of lecithin for use in the processes of the invention is preferably greater than 15 % by weight, more preferably greater than 25 % by weight, such as greater than 30 % by weight. For example, the phospholipid content of lecithin may range from 25 to 60 % by weight, such as from 30 to 40 % by weight. A commercially available preparation of lecithin suitable for use in the invention is Membranol-35 (Lipid Nutrition BV, Wormerveer, The Netherlands).

In a preferred embodiment, the phospholipid is present as an oil-in-water emulsion comprising lecithin.

The initial concentration of phospholipid in the total reaction volume preferably ranges from 0.5 to 25 % by weight, such as from 1 to 20 % by weight or from 2 to 15 % by weight, more preferably from 3 to 15 % by weight. Preferably greater than 15 % by weight of the phospholipid is phosphatidylcholine. For example, from 20 to 100 % by weight of the phospholipid is phosphatidylcholine, such as from 20 to 80 % by weight, from 25 to 60 % by weight, or from 25 to 40 % by weight.

In the process according to the second aspect of the invention, the phospholipid is hydrolysed in the presence of at least one surfactant.

Ionic or non-ionic surfactants can be conveniently used in the processes of the invention, with ionic surfactants being preferred. Suitable ionic surfactants include anionic surfactants, for example, a salt of a carboxylic acid, such as cholic acid. Suitable salts include, for example, food grade salts such as sodium salts. Sodium cholate is the preferred surfactant.

The initial concentration of the surfactant in the total reaction volume preferably ranges from 0.5 to 15 % by weight, such as from 1 to 10 % by weight, more preferably from 1.5 to 8 % by weight.

The serine used in the process according to the first aspect of the invention may be in either the D or L enantiomeric form or may be provided as a racemic mixture. L-serine is preferred.

The initial weight ratio of serine to phospholipid in the total reaction volume preferably ranges from 10:1 to 1:1, such as from 7:1 to 3:1.

The initial concentration of serine in the total reaction mixture preferably ranges from 5 to 70 % by weight, such as in the range of from 10 to 60 % by weight, more preferably from 20 to 50 % by weight, such as from 25 to 40 % by weight.

Phospholipase D from carrot is used in the processes of the invention. Typically, phospholipase D is present in the total reaction mixture in an amount greater than 0.0005 % by weight, most preferably greater than 0.001 % by weight or greater than 0.002 % by weight.

The processes of the invention are preferably carried out at a pH greater than 7. Most preferably, the processes are carried out at a pH in the range from 7 to 10, such as in the range of from 7 to 9. The desired pH may be obtained using known methods in the art, for example by the addition of any suitable base, such as ammonium hydroxide.

The processes of the invention may be carried out at any suitable temperature, such as at an elevated temperature in the range from 25 to 60°C, such as from 25 to 45°C. Typically, the processes are carried out at room temperature (i.e., at a temperature ranging from 21 to 25°C).

The phospholipid is preferably dispersed in an aqueous medium before admixing with the other reaction components. This may be achieved simply by mechanically stirring the phospholipid in water for a suitable amount of time.

To promote the dispersion of the phospholipid in the aqueous medium, the processes of the invention preferably comprise the step of preparing an aqueous mixture of the phospholipid and the surfactant. Thus, in the process according to the first aspect of the invention, the aqueous mixture of the phospholipid and the surfactant is preferably prepared prior to reacting with serine. In the process according to the second aspect of the invention, the aqueous mixture of the phospholipid and the surfactant is preferably prepared prior to hydrolysing the phospholipid. Advantageously, the aqueous mixture is prepared as a homogeneous mixture.

Preferably, the aqueous mixture of the phospholipid and the surfactant comprises from 2 to 50 % by weight phospholipid, such as from 5 to 40 % by weight or from 5 to 30 % by weight, more preferably from 10 to 20 % by weight phospholipid. The amount of surfactant in the aqueous mixture preferably ranges from 0.1 to 25 % by weight, such as from 3 to 20 % by weight, more preferably from 4 to 15 % by weight, such as from 5 to 12 % by weight.

The starting phospholipase D is preferably provided in an aqueous medium which typically comprises from 5 to 25 % by weight phospholipase D, most preferably from 10 to 20 % by weight phospholipase D.

Reacting the phospholipid with the serine in the process according to the first aspect of the invention is advantageously carried out in the presence of one or more metal ions. Similarly, hydrolysing the phospholipid in the process according to the second aspect of the invention is advantageously carried out in the presence of one or more metal ions. The one or more metal ions are typically mono- or divalent ions, for example, ions of calcium, potassium, sodium, magnesium and mixtures thereof. For example, a halide salt of a suitable metal ion as described above may be added to the reaction mixture. Particularly preferred are divalent calcium ions, which are preferably provided in the form of calcium chloride. The concentration of the one or more metal ions preferably ranges from 0.05 to 0.5 M.

The serine that is used in the process according to the first aspect of the invention is preferably provided in the form of an aqueous solution, which is then admixed with the other components of the reaction mixture. The amount of serine in the aqueous solution typically ranges from 50 to 80 % by weight, such as from 55 to 75 % by weight, more preferably from 60 to 70 % by weight. Advantageously, the aqueous solution of serine has a pH of greater than 7, such as a pH in the range of from 7 to 9. This can be achieved using known methods in the art, for example by the addition of any suitable base, such as ammonium hydroxide.

The reaction components used in the processes of the present invention may be admixed in any order.

In a preferred embodiment, the process according to the first aspect of the invention comprises the steps of:
(i) admixing the aqueous mixture of the phospholipid and the surfactant with the serine; and
(ii) adding phospholipase D to the reaction mixture produced in (i).

Preferably, the reaction mixture produced in step (i) is stirred (e.g., mechanically) before the phospholipase D is added to the reaction mixture in step (ii). Typically, the reaction mixture produced in step (ii) is mixed and then left to stand for a period of time, such as at least 1 hour (e.g., for at least 5 hours or at least 10 hours or at least 20 hours).

In an alternative embodiment of the process according to the first aspect, the phospholipase D may be admixed with the aqueous mixture of the phospholipid and the one or more surfactants prior to adding the serine to the reaction mixture.

The processes of the invention are preferably carried out in the complete or substantial absence of any added organic solvent (such as organic solvents including alcohols having from 1 to 12 carbon atoms, such as methanol, ethanol, n-propanol and isopropanol; ketones having from 3 to 8 carbon atoms and alkanes having from 1 to 12 carbon atoms (e.g., toluene, hexane and benzene); and aprotic organic solvents such as dimethylsulfoxide (DMSO), dimethylformamide, acetonitrile and N-methyl-pyrrolidone).

In the process for preparing phosphatidylserine, the amount, by weight of phosphatidylcholine in the starting phospholipid, of phosphatidylserine that is produced is preferably greater than 10 %, such as greater than 12 % or 15 %, most preferably greater than 18 % or 20 %.

In the process for preparing phosphatidic acid, the amount, by weight of phosphatidylcholine in the starting phospholipid, of phosphatidic acid that is produced is preferably from 20 to 50%, such as from 25 to 45%.

Phosphatidylserine may be recovered from the reaction mixture by filtration. The filtrate is optionally washed to remove the serine and the metal salts. The phosphatidylserine may optionally be further purified, if required.

### Phospholipase D

Phospholipase D may be isolated from carrot using methods known in the art.

A particularly preferred method for isolating phospholipase D from carrot comprises the following steps:
(a) providing a liquid extract from carrot;
(b) combining the liquid extract with a precipitating agent comprising phospholipids, mono-, di- or tri-glycerides or a mixture thereof in admixture with at least one surfactant, to produce a suspension comprising a supernatant and a precipitate; and
(c) separating and collecting the precipitate from the suspension produced in (b).

The liquid extract is preferably provided in the form of a juice or similar liquid preparation. The liquid extract may not be fully liquid and may comprise some solids. Typically, the carrot is cooled to a temperature below room temperature (i.e., below 25°C), such as at a temperature in the range of from 1 to 10°C or from 2 to 7°C, washed and chopped. The juice is then extracted from the chopped preparation by methods known in the art, for example by using a domestic or commercial juice extractor. In some cases a proportion of the original phospholipase D content of the carrot will be left in the solid wastes after the initial removal of the juice. Optionally, additional phospholipase D may be extracted from these solid wastes by the addition of water and re-processing through the juice extractor. The person skilled in the art will appreciate that the liquid extract obtained from each of the above extraction steps may be pooled to provide the liquid extract for use in step (a).

Preferably, the process as described above, for the preparation of the liquid extract for use in step (a), is performed at a temperature below room temperature (i.e., below 25°C), such as at a temperature of from 1 to 10°C or from 2 to 7°C. The resulting supernatant is collected and, if it is to be stored, is preferably frozen in liquid nitrogen and stored at around -70°C.

Routine methods for the detection of the phospholipase D activity may be used to monitor the efficiency of the above extraction process to ensure that adequate quantities of phospholipase D are extracted from the carrot.

Using the above extraction method, it is possible to obtain a high yield of liquid extract from the carrot. For example, 0.6 to 0.67 litres of liquid extract may be extracted from 1 kg of raw carrot.

It will be appreciated that the liquid extract produced by the above extraction method may contain residues. For example, crude carrot extracts may contain floating lipids saturated with carotin. Hence, in a preferred embodiment of the invention, the liquid extract is subjected to a clarification process prior to step (b) to produce a clarified liquid extract. Preferably, therefore, the liquid extract is in the form of a clarified liquid extract.

The clarification process typically comprises contacting the liquid extract with a clarifying agent comprising one or more metal ions to produce a suspension comprising a clarified liquid extract and a precipitate. The resulting clarified liquid extract is then separated and collected from the suspension.

Thus, in a preferred embodiment, the method for isolating the phospholipase D from carrot comprises the following steps prior to step (b):
A. contacting the liquid extract of step (a) with a clarifying agent comprising one or more metal ions to produce a suspension comprising a clarified liquid extract and a precipitate; and
B. separating and collecting the clarified liquid extract from the suspension produced in step (A).

Typically, the one or more metal ions in the clarifying agent are mono- or divalent ions, for example, ions of calcium, sodium, potassium, magnesium and mixtures thereof. For example, a halide salt of a suitable metal ion as described above may be used. Divalent calcium ions are preferred, which are typically provided in the form of calcium chloride.

The concentration of the one or more metal ions in step (A) preferably ranges from 20 to 60 mM, such as from 30 to 50 mM or from 35 to 45 mM.

The contacting of the liquid extract of step (a) with a clarifying agent in step (A) is preferably carried out at a temperature below room temperature, i.e., below 25°C, such as at a temperature of from 1 and 20°C, more preferably from 1 to 15°C, such as from 2 to 10°C or from 2 to 7°C. Upon contacting, the mixture is ideally mixed (e.g., by mechanical stirring).

Preferably, step (A) is carried out at a pH in the range of from 4 to 9. Most preferably, the pH is greater than 7, such as in the range of from 7 to 9 or from 7 to 8. The desired pH may be obtained using methods known in the art, for example by the addition of any suitable base, such as sodium hydroxide or ammonium hydroxide.

In a preferred embodiment of step (A), after the liquid extract of step (a) and the clarifying agent have been contacted and the pH has been adjusted as desired, the temperature of the mixture is preferably dropped to around 0°C and the mixture left for a sufficient amount of time (for example, about 20 minutes) for the precipitate to form.

The clarified liquid extract produced in step (A) may be separated from the precipitate using known methods in the art, such as by centrifugation and/or filtration. For example, in step (B) the suspension may be filtered and the filtrate centrifuged at around 9000 rpm for about 15 minutes.

During step (B) the temperature is preferably maintained below room temperature, such as at a temperature of from 1 and 20°C, more preferably from 1 to 15°C, such as from 2 to 10°C or from 2 to 7°C.

Typically, steps (A) and (B) result in minor reductions in the amount of phospholipase D in the liquid extract. For example, the clarification process may produce less than 10 % by weight reduction in the amount of phospholipase D in the clarified liquid extract compared to the unclarified liquid extract.

The precipitating agent is preferably in the form of an aqueous emulsion. Preferably, the precipitating agent comprises phospholipids. The phospholipids may be derived from synthetic or natural sources. Representative examples of phospholipids that may be present in the second precipitating agent include phosphatidylcholine, phosphatidylethanolamine, phosphatidylinositol, phosphatidyl glycerol, sphingomyelin and mixtures thereof. The preferred phospholipid is phosphatidylcholine.

The precipitating agent used in the method for isolating phospholipase D from carrot may be derived from naturally occurring substances obtained from animal and vegetable sources such as lecithin, cephalin, and sphingomyelin. Preferably the precipitating agent is derived from lecithin.

Lecithin contains a mixture of glycolipids, triglycerides, and phospholipids (e.g., phosphatidylcholine, phosphatidylethanolamine, and phosphatidylinositol) and may be derived from natural sources or synthetic sources. Preferably, lecithin for use in the preparation of the precipitating agent is obtained from animal or vegetable sources, such as from soy beans, egg yolks or rapeseed, using conventional processing methods. A suitable commercially available preparation of lecithin includes, for example, Membranol-35 (Lipid Nutrition B.V., Wormerveer, The Netherlands).

Preferably, the phospholipid content of lecithin for use in the preparation of the precipitating agent is greater than 15 % by weight, more preferably greater than 25 % by weight, such as greater than 30 % by weight or greater than 50 % by weight.

In a preferred embodiment, the amount by weight of phospholipids in the precipitating agent ranges from 0.1 to 7 % by weight, such as from 0.5 to 4 % by weight, more preferably from 0.8 to 3 % by weight, or from 0.8 to 2 % by weight.

The amount of phospholipids in step (b), based on the total reaction volume, is preferably in the range of from 0.05 to 1 % by weight, more preferably from 0.1 to 0.5 % by weight, such as from 0.1 to 0.3 % by weight.

The phosphatidylcholine content, by weight of the phospholipids present in the precipitating agent, is preferably greater than 15 % by weight. For example, the phosphatidylcholine content may range from 20 to 100 % by weight, such as from 20 to 80 % by weight, from 25 to 60 % by weight, or from 25 to 40 % by weight of the phospholipids in the precipitating agent.

Optionally, the precipitating agent further comprises one or more metal ions. The one or more metal ions are preferably mono- or divalent ions, for example, ions of sodium, potassium, calcium, magnesium and mixtures thereof. For example, the precipitating agent may comprise an aqueous solution of a halide salt of a suitable metal ion. Divalent calcium ions are preferred, which are typically provided in the form of calcium chloride.

The concentration of the one or more metal ions in step (b) preferably ranges from 20 to 60 mM, such as from 30 to 50 mM or from 35 to 45 mM.

In the preferred embodiment, in which the liquid extract is subjected to a clarification process (i.e., steps (A) and (B)), it will be appreciated that one or more metal ions will already be present in the clarified liquid extract and, as a consequence, it is not necessary for the precipitating agent to comprise one or more metal ions. However, due to the fact that some calcium ions may be bound to the precipitate in step (B), in a preferred embodiment, the precipitating agent comprises one or more metal ions, preferably in an amount such that the concentration of the metal ions in step (b) falls within the preferred concentration ranges as specified above.

The precipitating agent further comprises at least one surfactant. Ionic or non-ionic surfactants are preferably present in the second precipitating agent, with ionic surfactants being particularly preferred. Suitable ionic surfactants include anionic surfactants, for example, a salt of a carboxylic acid, such as cholic acid, a salt of a (C₄ to C₂₄) linear alkyl sulphate, such as sodium dodecyl sulphate, or a mixture thereof. Suitable salts of cholic acid include, for example, food grade salts such as sodium salts. Sodium dodecyl sulphate is the preferred surfactant.

The amount of surfactant in the precipitating agent is preferably in the range of from 0.05 to 5 % by weight, more preferably from 0.1 to 3 % by weight or from 0.2 to 1.5 % by weight.

The amount of surfactant in step (b), based on the total reaction volume, preferably ranges from 0.01 to 3 % by weight, more preferably from 0.05 to 1.0 % by weight, most preferably from 0.08 to 0.16 % by weight.

In a preferred embodiment, the precipitating agent is prepared as an aqueous emulsion. Advantageously, the precipitating agent is prepared as a homogeneous mixture. This may be achieved, for example, simply by mechanically stirring the precipitating agent in an aqueous medium for a suitable amount of time. The presence of the surfactant may help to promote the dispersion of the components of the precipitating agent within the aqueous medium.

The precipitating agent preferably has a pH greater than 7, preferably a pH of from 7 to 9, such as from 7 to 8.5 or from 7.2 to 8. This pH can be achieved using known methods in the art, for example by the addition of any suitable base, such as sodium hydroxide or ammonium hydroxide.

Step (b) of the process is preferably performed at a temperature below room temperature, i.e., at a temperature below 25°C. Most preferably, step (b) is performed at a temperature of from 0 to 15°C, such as from 1 to 10°C or from 1 to 5°C. Typically, the pH of the mixture in step (b) is greater than 7 (for example, at a pH in the range of from 7 to 9, most preferably, in the range of from 7 to 7.5).

Without wishing to be bound by theory, it is believed that during step (b) phospholipase D in the liquid extract is adsorbed, complexed or otherwise associated onto the precipitate formed from the one or more metal ions and the surfactant. For example, when the liquid extract is contacted with the precipitating agent comprising sodium dodecyl sulphate (SDS) in the presence of calcium chloride, it is believed that phospholipase D adsorbs onto the resulting calcium/SDS precipitate.

Step (b) is preferably performed to the stage when at least 30 % by weight or at least 40 % by weight of the phospholipase D in the liquid extract is recovered in the precipitate. More preferably at least 50 % by weight, most preferably at least 60 % by weight or at least 66 % by weight of the phospholipase D in the liquid extract is recovered in the precipitate.

Although the rate of formation of the precipitation or recovery of the phospholipase D in the precipitate may vary depending on the temperature and other process variables, step (b) is preferably carried out for a period of at least 5 minutes, more preferably for a period greater than 30 minutes or 1 hour. Most preferably, step (b) is carried out for a period of from 30 minutes to 30 hours, such as from 1 to 24 hours, from 1 to 10 hours, from 1 to 5 hours or from 1.5 to 2.5 hours.

The precipitate produced in step (b) may be separated from the supernatant using known methods in the art, such as by centrifugation and/or filtration. For example, in step (c) the suspension may be centrifuged at around 6000 rpm for about 15 minutes.

During step (c) the temperature is preferably maintained below room temperature, such as in the range of from 1 and 20°C, more preferably from 1 to 15°C, such as from 2 to 10°C or from 2 to 7°C.

Typically, the collected precipitate is subsequently dried. Drying the precipitate may be carried out using known methods in the art. In a preferred embodiment, the precipitate is suspended in water, frozen (in liquid nitrogen, for example) and lyophilized.

In order to improve the shelf-life or preserve the activity of the phospholipase D, the phospholipase D is preferably lyophilized in the presence of potassium chloride, histidine-HCl, calcium chloride, sodium acetate or mixtures thereof, typically, at a pH of from 5 to 7. Most preferably, the phospholipase D produced by the invention is lyophilized in the presence of potassium chloride and sodium acetate, preferably at a pH of from 5 to 6.

The amount of phospholipase D produced, based on the total volume of liquid extract, is preferably greater than 0.2 % by weight, such as greater than 0.3 % by weight or greater than 0.5 % by weight, most preferably greater than 0.7 % by weight.

The following non-limiting examples illustrate the invention. In the examples and throughout the specification, all percentages, parts and ratios are by weight unless indicated otherwise.

### Examples

### Example 1

### Preparation of PS

Phosphatidylserine (PS) was synthesised from Membranol-35 (Lipid Nutrition B.V., Wormerveer, The Netherlands) and serine catalysed by phospholipase D (PLD) obtained from carrot (see Examples 2A and 3 below)

The following solutions were prepared:
A. Preparation of membranol-cholate emulsion
   15 ml of 10% sodium cholate was mixed with 2.5 ml of water and 7.5 g of membranol and shaken for about 1 hour until a homogenous mixture was formed.
B. Preparation of serine suspension
   200 mg of dry serine powder was mixed with 260 µl of water and 40 µl NH₄OH (conc.).
C. Preparation of PLD suspension
   32 mg of the lyophilized PLD produced by Example 2A below was mixed with 200 µl of water.

### Transesterification reaction

200 µl of suspension B was added to 250 mg of preparation A. The mixture was well vortexed. After that 20 µl of enzyme preparation C was added and the mixture was well mixed. 20 µl of 6M CaCl₂ solution was then added. The reaction mixture was well mixed and then left without mixing at room temperature for 22 hours.

### Analysis of the reaction mixture

1) The reaction mixture was briefly extracted by vortexing with 1 ml of methanol-chloroform (1:2 v/v). Phases were separated by brief centrifugation; organic (lower) phases were 10-fold diluted with methanol-chloroform (1:2 v/v). 5 µl aliquots of the organic extracts and a control PS solution were applied to twin thin-layer silica gel plates on one large sheet.
2) The chromatography was performed in chloroform/methanol/water, 100:50:3.
3) After drying the chromatography sheet was divided in two parts (plates). One was stained by 1% solution of ninhydrin in acetone and then heated at about 50°C for about 10 min until purple spots appeared for PS. The other plate was stained by CuSO₄-H₃PO₄ (dipping in 0.63 M CuSO₄/0.85 M H₃PO₄ water solution) and then heated at 110°C for about 30 min until dark-grey spots appeared for lipids.

### Quantitative determination of phospholipids and phosphatidylserine (PS)

The method described by Vaskovsky et al, "A universal reagent for phospholipid analysis" J. Chromat. (1975), vol. 114, N 1, pages 129-141 was followed.

The weight percent of phospholipids in the reaction mixture (based on the weight of phosphatidylcholine (PC) in Membranol-35) was as follows: PS about 20-25 %; PC about 40-45%; and phosphatidic acid (PA) about 35%.

### Example 2A

### PLD preparation from carrot by Ca-SDS-Membranol-35 enzyme precipitation

3.9 kilograms of carrot were firstly chilled to 4°C, washed and chopped. A sap was obtained with juice extractor Braun MP 80 at 4°C. Additional PLD that remained in the hard wastes (residues) of the carrot was recovered by re-processing the hard wastes in the juice extractor. Altogether 2400 ml carrot juice and 1500 g wastes were received. The juice was filtered through 4 layers of gauze. The filtrate was centrifuged at 9000 rpm (Beckman J-21 centrifuge, JA-14 rotor) for 15 min at 4°C. After centrifugation 2300 ml supernatant was collected and frozen in liquid nitrogen and stored at -70°C. The supernatant was bright yellow.

Carrot juice was quickly defrosted with lukewarm water, and then placed in an ice water bath. To 300 ml of defrosted juice, 14 ml of 1 M CaCl₂ was slowly added under constant mixing up to final concentration of 40 mM. The pH of the mixture was then carefully adjusted to pH 7.4 by adding of 1 M NH₄OH and exposed for 20 min at 0°C. The formed precipitate was separated by centrifugation at 6000 rpm (Beckman J-21 centrifuge, JA-14 rotor) for 15 min at 4°C. The supernatant (clarified juice) was collected.

To 160 ml clarified juice, 16 ml SDS-Membranol-35 emulsion (see Example 3 for the preparation of the emulsion) was added and the mixture was well mixed. 8 ml of 1 M CaCl₂ was added in small portions (1 ml). Under stirring 5 ml 1 N NH₄OH solution was added drop wise until a pH of about 7.15 was reached. The whole mixture was kept in ice bath for approximately 2 hours. The formed precipitate was separated by centrifugation at 6000 rpm (Beckman J-21 centrifuge, JA-14 rotor) for 15 min at 4°C. The pellet was suspended in about 5 ml of water, frozen in liquid nitrogen and lyophilized.

After lyophilization 1.29 g lyophilized preparation was received. From 1 ml carrot juice approximately 8 mg powder was obtained.

### Example 2B

### Precipitation of PLD by addition of CaCl₂, then SDS

To 2.4 g of water solution of sodium dodecyl sulphate (SDS) 0.15 ml 1 N NaOH were added while stirring. Then water was added to 40 ml total to make an SDS-solution.

To 16 ml of clarified carrot juice, 0.8 ml 1 M CaCl₂ was added dropwise then 1.6 ml of the SDS-solution was added and the mixture was well mixed. Under stirring a few drops of 1 N NH₄OH solution were added drop wise until a pH 7.16 was reached. The whole mixture was kept in an ice bath for approximately 1 hour. The formed precipitate was separated by centrifugation at 3500 rpm (Jouan-Paris K-63F) for 10 min at 4°C. The pellet was suspended in about 0.5 ml of water, frozen in liquid nitrogen and lyophilized.

After lyophilization 17.2 mg lyophilized preparation was received. From 1 ml carrot juice approximately 1.1 mg of powder was obtained with the specific activity about 48.9x10⁻² µmol per minute per 1 mg of preparation.

### Example 3

### Preparation of SDS-Membranol-35 emulsion

To 1.2 ml Membranol-35 (Lipid Nutrition B.V., Wormerveer, The Netherlands), 0.15 ml 1 N NaOH and 2.4 ml 10% water solution of sodium dodecyl sulfate (SDS) was added while stirring. Then water in small portions (5 times x 1 ml) was added under vigorous stirring until homogeneous mixture was formed. Then water was added to 40 ml total and the mixture was stirred for about 30 min on a shaker.

### Examples 4 and 5

### Formation of PS under different reaction conditions

### A. Preparation of the Lecithin/Serine emulsion

Lecithin (1.0 g) was placed in a glass vial with 3 ml of water and 1 ml of 1 N NaOH solution. The components were intensively stirred with magnetic stirrer for about 0.5 h at RT until a homogenous mixture was formed. Then 2 g of serine powder was added and the mixture was intensively stirred with the magnetic stirrer at RT for about 1 h.

### B. 1 M Sodium-acetate buffer pH 6.0 preparation

To 13.6 g of Na(CH₃COO) 3H₂O ("Reakhim") water was added to obtain the total volume 100 ml. Glacial acetic acid was diluted 17 times. To Na (CH₃COO) solution the diluted acetic acid was added while mixing until a pH of 6.0 was reached.

### C. Preparation of the stock reaction mixture

Preparation A (1.0 g) was placed in a glass vial with 0.5 ml of preparation B. The components were intensively stirred with magnetic stirrer for about 0.5 h at RT until a homogenous mixture was formed. Then 2 mg of the PLD-Ca/Membranol precipitate was added and the mixture was intensively stirred with the magnetic stirrer for about 2 min.

### D. Preparation of 2 M CaCl₂_solution

14.7 g of CaCl_{2·}2H₂O was dissolved in water, pH was adjusted to about 7 (indicator paper) by addition of 1 N NaOH solution. The volume of solution was adjusted to 50 ml.

### Comparative Example 1

### Mixture C - control at 50°C

0.2 ml of preparation C was placed in plastic 2.2 ml Eppendorf tube. The tube was sealed, the mixture was thoroughly vortexed and left without stirring at 50°C. Aliquot for analysis was picked up at 1 h and analyzed by TLC.

### Comparative Example 2

### To Mixture C - CaCl₂ added

30 µl of H₂O, 30 µl of preparation D and 0.2 ml of preparation C was placed in plastic 2.2 ml Eppendorf tube. The tube was sealed, the mixture was thoroughly vortexed and left without stirring at 50°C. Aliquot for analysis was picked up at 1 h and analyzed by TLC.

### Example 4

### To Mixture C - CaC₂ and sodium cholate added

30 µl of H₂O, 6 mg of the sodium cholate powder and 0.2 ml of preparation C was placed in plastic 2.2 ml Eppendorf tube. The tube was sealed, the mixture was thoroughly vortexed. Then 30 µl of preparation D was added, the tube was sealed, the mixture was thoroughly vortexed and left without stirring at 50°C. Aliquot for analysis was picked up at 1 h and analyzed by TLC.

The above examples were repeated using Membranol instead of lecithin.

The results of the experiments are given in the following table.

| Example | Substrate | Additives | Product phospholipid composition,% | | | | |
|---|---|---|---|---|---|---|---|
| | | | PE | PA | PC | PS | PS/PA |
| Comparative Example 1 | Lecithin | None | 35 | 16 | 47 | 2 | 0,12 |
| Comparative Example 2 | Lecithin | CaCl₂ | 15 | 52 | 18 | 15 | 0,28 |
| Example 4 | Lecithin | CaCl₂, cholate | 13 | 46 | 23 | 18 | 0,38 |
| Comparative Example 3 | Membranol | None | 26 | 5 | 67 | 1 | 0,25 |
| Comparative Example 4 | Membranol | CaCl₂ | 16 | 38 | 32 | 14 | 0,38 |
| Example 5 | Membranol | CaCl₂, cholate | 6 | 51 | 13 | 30 | 0,58 |

## Claims

1. A process for preparing phosphatidylserine comprising reacting a phospholipid with serine in the presence of water, a phospholipase D from carrot and at least one surfactant.

2. A process for preparing phosphatidic acid comprising hydrolysing a phospholipid in the presence of water, a phospholipase D from carrot and at least one surfactant.

3. A process as claimed in any of the preceding claims, wherein the phospholipid is present as an oil-in-water emulsion comprising lecithin.

4. A process as claimed in any one of Claims 1 to 3, wherein the surfactant is an anionic surfactant

5. A process as claimed in any one of Claims 1 to 4, wherein the surfactant is a salt of a carboxylic acid.

6. A process as claimed in any one of Claims 1 to 5, wherein the surfactant is a salt of cholic acid.

7. A process as claimed in any one of Claims 1 to 6, wherein the surfactant is sodium cholate.

8. A process as claimed in any one of the preceding claims, which is carried out at a pH of greater than 7.

9. A process as claimed in any one of Claims 1 or 3 to 8, wherein reacting the phospholipid with the serine is carried out in the presence of one or more metal ions.

10. A process as claimed in any one of Claims 1 or 3 to 9, which comprises the step of preparing an aqueous mixture of the phospholipid and the surfactant prior to reacting with serine.

11. A process as claimed in any one of Claims 1 or 3 to 10, wherein the serine is provided in the form of ah aqueous solution.

12. A process as claimed in Claim 11, wherein the aqueous solution is at a pH of greater than 7.

13. A process as claimed in any one of Claims 10 to 12, wherein the process comprises the steps of:
(i) admixing the mixture of the phospholipid and the surfactant with the serine: and
(ii) adding phospholipase D to the reaction mixture produced in step (i).

## Patentansprüche

1. Verfahren zur Herstellung von Phosphatidylserin umfassend das Reagieren eines Phospholipids mit Serin in Gegenwart von Wasser, einer Phospholipase D aus Karotte und mindestens einem Tensid.

2. Verfahren zur Herstellung von Phosphatidsäure umfassend das Hydrolysieren eines Phospholipids in Gegenwart von Wasser, einer Phospholipase D aus Karotte und mindestens einem Tensid.

3. Verfahren nach einem der vorhergehenden Ansprüche, in welchem das Phospholipid als Öl-in-Wasser-Emulsion umfassend Lecithin vorliegt.

4. Verfahren nach irgendeinem der Ansprüche 1 bis 3, in welchem das Tensid ein anionisches Tensid ist

5. Verfahren nach irgendeinem der Ansprüche 1 bis 4, in welchem das Tensid ein Salz einer Carbonsäure ist.

6. Verfahren nach irgendeinem der Ansprüche 1 bis 5, in welchem das Tensid ein Salz einer Cholsäure ist.

7. Verfahren nach irgendeinem der Ansprüche 1 bis 6, in welchem das Tensid ein Natriumcholat ist.

8. Verfahren nach irgendeinem der vorhergehenden Ansprüche, das bei einem pH-Wert größer als 7 durchgeführt wird.

9. Verfahren nach irgendeinem der Ansprüche 1 oder 3 bis 8, in welchem das Reagieren des Phospholipids mit dem Serin in Gegenwart von einem oder mehreren Metallionen durchgeführt wird.

10. Verfahren nach irgendeinem der Ansprüche 1 oder 3 bis 9, das den folgenden Schritt enthält: die Zubereitung eines wasserhaltigen Gemisches des Phospholipids und des Tensids vor dem Reagieren mit Serin.

11. Verfahren nach irgendeinem der Ansprüche 1 oder 3 bis 10, in welchem das Serin in Form einer wäßrigen Lösung vorgesehen ist.

12. Verfahren nach Anspruch 11, in welchem die wäßrige Lösung einen pH-Wert über 7 aufweist.

13. Verfahren nach irgendeinem der Ansprüche 10 bis 12, wobei das Verfahren die folgenden Stufen enthält:
(i) das Beimengen des Gemisches des Phospholipids und des Tensids mit dem Serin; und
(ii) das Addieren von Phospholipase D zum in der Stufe (i) erzeugten Reaktionsgemisch.

## Revendications

1. Procédé de préparation de phosphatidylsérine comprenant la réaction d'un phospholipide avec une sérine en présence d'eau, d'une phospholipase D issue de la carotte et d'au moins un tensioactif.

2. Procédé de préparation d'acide phosphatidique comprenant l'hydrolyse d'un phospholipide en présence d'eau, d'une phospholipase D issue de la carotte et d'au moins un tensioactif.

3. Procédé selon l'une quelconque des revendications précédentes, dans lequel le phospholipide est présent sous la forme d'une émulsion huile dans l'eau comprenant une lécithine.

4. Procédé selon l'une quelconque des revendications 1 à 3, dans lequel le tensioactif est un tensioactif anionique.

5. Procédé selon l'une quelconque des revendications 1 à 4, dans lequel le tensioactif est un sel d'acide carboxylique.

6. Procédé selon l'une quelconque des revendications 1 à 5, dans lequel le tensioactif est un sel d'acide cholique.

7. Procédé selon l'une quelconque des revendications 1 à 6, dans lequel le tensioactif est le cholate de sodium.

8. Procédé selon l'une quelconque des revendications précédentes, qui est mis en oeuvre à un pH supérieur à 7.

9. Procédé selon l'une quelconque des revendications 1 ou 3 à 8, dans lequel la réaction du phospholipide avec la sérine est mise en oeuvre en présence d'un ou de plusieurs ions métalliques.

10. Procédé selon l'une quelconque des revendications 1 ou 3 à 9, qui comprend l'étape de préparation d'un mélange aqueux du phospholipide et du tensioactif avant réaction avec la sérine.

11. Procédé selon l'une quelconque des revendications 1 ou 3 à 10, dans lequel la sérine est utilisée sous la forme d'une solution aqueuse.

12. Procédé selon la revendication 11, dans lequel la solution aqueuse est à un pH supérieur à 7.

13. Procédé selon l'une quelconque des revendications 10 à 12, dans lequel le procédé comprend les étapes suivantes :
(i) ajout du mélange du phospholipide et du tensioactif à la sérine ; et
(ii) ajout de la phospholipase D au mélange réactionnel obtenu à l'étape (i).
